# EUROPEAN PATENT SPECIFICATION

(11) **EP 2 869 800 B1**
(45) Date of publication and mention of the grant of the patent: **18.11.2020**
(21) Application number: 13812928.3
(22) Date of filing: 01.07.2013
(51) Int. Cl.: A61F 7/00, A61F 7/10, A61H 39/06, A61F 13/02, A61F 7/02, A61F 13/00

(54) **PRESSURE ULCER MANAGEMENT PAD**
DEKUBITUSBEHANDLUNGSKISSEN
PROTECTION DE PRISE EN CHARGE D'UNE PLAIE DE PRESSION

(30) Priority: 05.07.2012 US 201261668259 P
(43) Date of publication of application: 13.05.2015
(73) Proprietor: Stryker Corporation, Kalamazoo, MI 49002 (US)
(72) Inventor: LAFLECHE, Patrick, Kalamazoo, Michigan 49009 (US)
(74) Representative: FRKelly
(86) International application number: PCT/US2013/048916
(87) International publication number: WO 2014/008182

(56) References cited:
- EP-A1- 1 142 515
- EP-A1- 1 219 211
- WO-A1-00/03666
- WO-A1-93/09737
- GB-A- 2 447 287
- US-A- 3 681 797
- US-A- 5 486 206
- US-A- 5 643 336
- US-A- 5 837 002
- US-A- 5 894 615
- US-A1- 2011 010 850
- US-A1- 2012 065 715
- US-B1- 6 402 775
- US-B2- 6 699 266
- US-B2- 6 859 967

## Description

### TECHNICAL FIELD AND BACKGROUND OF THE INVENTION

The present invention relates to a pad or overlay, and more particularly, a pad or overlay that is particularly suitable for use under a patient or invalid supported on a surface, such as on a cushion or mattress, in a hospital or other patient care facility, including long term care facilities or the like.

When patients are hospitalized or bedridden for any significant amount of time, patients can develop pressure sores or ulcers. These pressure sores or ulcers can be exacerbated by the patient's own poor circulation, such as in the case of diabetic patients, but typically form as a result of prolonged immobility, which allows the pressure exerted on the patient's skin from the mattress to decrease circulation in the patient's tissue. In addition to reducing circulation in the patients' tissue, lack of mobility can also cause heat and moisture build-up at the point of contact with the mattress. Heat contributes to the moisture build-up, which can cause maceration in the skin-which makes the skin more permeable and vulnerable to irritants and stresses, such as stresses caused by pressure or by shear, for example when a patient is moved across a mattress. It has been found with the same pressure an increase in skin temperature can cause an increase in tissue damage.

To address some of these issues, some beds incorporate a low air loss system, which directs air to the patient/mattress interface to reduce moisture. However, these systems tend to position dependent and are not adjustable.

Accordingly there is a need for surface that can at least reduce the heat build up that can occur at the interface between the supporting surface and the patient's skin. GB2447287 which is considered to be the closest prior art; discloses a cooling sheet comprising a gel having a closed loop cooling circuit embedded in it.

### SUMMARY OF THE INVENTION

The present invention provides a temperature management pad that cools a patient's skin when the patient is lying on top of the pad. as defined in independent claim 1. Preferred embodiments are disclosed in the dependent claims. Optionally, the pad is independent of and sized to be smaller in one or more dimensions than the underlying support surface so that it can be moved for better alignment with the areas of the patient's body that are more vulnerable, which are patient dependent.

In one disclosure, a temperature management pad includes a patient facing side, a cushioning layer, and a cooling layer. For example the cooling layer may comprise a fluid conduit for circulating fluid through the pad across the cushioning layer adjacent the patient facing side, which is a configured so that when a patient is lying on the patient facing side of the pad and over the fluid conduit the conduit will not be occluded by the weight of the patient.

In another disclosure a temperature management pad includes a patient facing side, a cushioning layer supporting the patient facing side above a surface, and a fluid conduit between the patient facing side and the cushioning layer for circulating fluid, such as cooled liquid, including cooled water and optionally water with fluorocarbons, through the pad across the cushioning layer. The cushioning layer is a configured so that when a patient is lying on the patient facing side of the pad and over the fluid conduit the conduit will not be occluded by the weight of the patient.

In yet another disclosure, a temperature management pad includes a patient facing side, a cushioning layer supporting the patient facing side above a support surface, and a fluid conduit formed between the patient facing side and the cushioning layer for circulating fluid, such as cooled liquid, through the pad across the cushioning layer. The cushioning layer is adapted to at least reduce (1) shear between the patient facing layer and the support surface on which the pad is supported or (2) pressure on the patient's skin.

According to another disclosure, a temperature management pad includes a patient facing side, a gel cushioning layer supporting the patient facing side above a support surface, a fluid conduit between the patient facing side and the cushioning layer for circulating cooled fluid through the pad across the cushioning layer. The gel cushioning layer has a continuous upper surface for supporting the patient facing side, with channels formed therein for locating the fluid conduit.

In any of the above pads, the patient facing side is formed by a patent facing layer, with the fluid or liquid conduit formed in the patient facing layer. For example, the patient facing layer may be formed by a film, such as a flexible polymeric film, including a polyester and polyether polyurethane film, a polyvinylchoride (PVC) film, a neoprene film, or a polyethylene film. Optionally, the patient facing layer is formed by at least two films, which are joined together to form the fluid or liquid conduit.

In any of the above pads, the cushioning layer includes a support surface facing side and a plurality of walls at the support facing side for supporting the pad on the support surface. For example, the walls may be configured to locally buckle under a pressure of a predetermine magnitude.

In a further aspect, the walls intersect to form a grid.

In yet another aspect, in any of the pads, the cushioning layer may comprise foam, gel, or a plurality of air bladders.

In any of the above pads with a gel layer, the gel layer may comprise a structural gel or a flowable gel, including a flowable gel with a plurality of spherical bodies.

According to yet another disclosure, a temperature management pad includes a gel cushioning layer having a continuous upper surface for supporting a patient above a support surface and a cooling layer, such as a fluid conduit.

In one aspect, the gel cushioning layer includes a support surface facing side and a plurality of walls at the support facing sides for supporting the pad on the support surface. For example, the walls may be configured to locally buckle under a pressure of a predetermine magnitude.

In any of the above pads, the cushioning layer may include a plurality of channels at its continuous upper surface for locating a plurality of fluid conduits. Further, in any of the above pads, the pad may include an inlet conduit for fluid communication with fluid supply tubing and an outlet conduit, with the inlet conduit directing fluid to one or more of the plurality of the fluid conduits, and the outlet conduit receiving fluid from one or more of the plurality of the fluid conduits for discharge or recirculation through the pad.

In yet another disclosure, a method of reducing pressure ulcer formation on a patient's skin includes supporting a patient on a mattress or cushion, suspending the patient's skin above the surface of the mattress or cushion, and cooling the interface between the patient's skin and the mattress or cushion.

For example, in any of the above pads or methods, the temperature of the cooled fluid or cooling component or device may be less than 30 degrees Celsius, less than 29 degrees Celsius, less than 28 degrees Celsius, less than 27 degrees Celsius, less than 26 degrees Celsius, less than 25 degrees Celsius, less than 24 degrees Celsius, less than 23 degrees Celsius, less than 22 degrees Celsius, less than 21 degrees Celsius or less than 20 degrees Celsius, including as low as 15 degrees Celsius. Optionally, the cooled fluid may be in a range of about 30 degrees Celsius to 15 degrees Celsius, in a range of about 28 degrees Celsius to 18 degrees Celsius, or in a range of about 26 degrees Celsius to 21 degrees Celsius.

According to another aspect, in any of the above pads or methods, the cooled fluid or cooling component or device induces a minimum temperature change at the skin of the patient of about 10 degrees Celsius, of about 8 degrees Celsius, of about 5 degrees Celsius, of about 3 degrees Celsius.

In a further aspect, the method may also include reducing pressure and/ or shear between the patient's skin and the mattress or cushion.

Accordingly, the pad that is configured to cool a patient's skin while not significantly, if at all, interfering with the pressure redistribution by, shear reduction by, and/or immersion of a patient into the underlying support surface.

These and other objects, advantages, purposes, and features of the invention will become more apparent from the study of the following description taken in conjunction with the drawings.

### DESCRIPTION OF THE FIGURES

FIG. 1 is a perspective view of a pad of the present invention on a bed supporting a patient;
FIG. 2 is a top plan view of the pad of FIG. 1 with the top layer removed to show a flow path through the pad;
FIG. 3 is a bottom plan view of the pad of FIG. 2; and
FIG. 4 is a cross-section view through the pad.

### DETAILED DESCRIPTION OF THE PREFERRED EMBODIMENTS

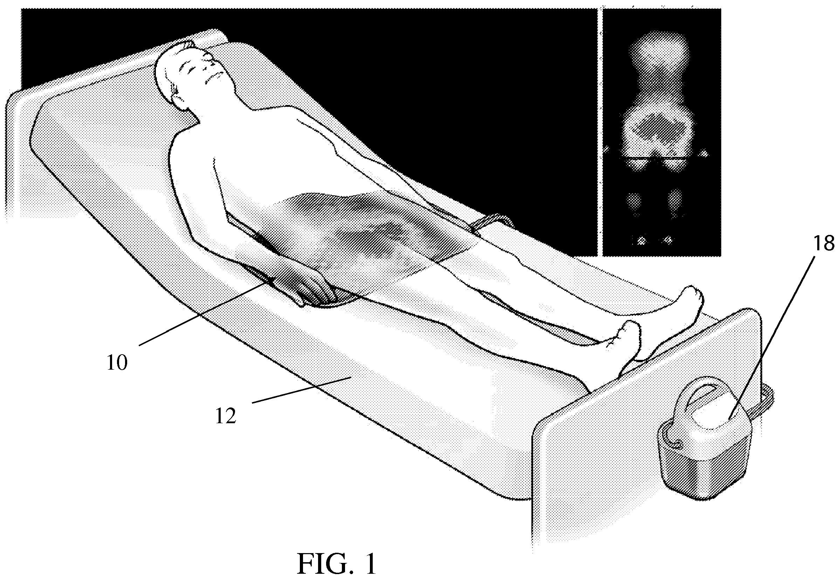

Referring to FIG. 1, the numeral 10 generally designates a pad of the present invention that is configured for use on a mattress 12 and under a patient P, for example under the sacral region of a patient, and to circulate cooling fluid under the patient to cool the skin of the patient. While illustrated as being used under the sacral region of a patient, it should be understood that pad 10 may also be used (and optionally reconfigured) for use under other regions of a patient's body, such as the heels. As will be more fully described below, pad 10 may be configured to provide shear reduction and/or pressure redistribution that supplements the shear reduction and/or pressure redistribution characteristics of an underlying mattress, such as a mattress for a hospital bed. For details of a suitable mattress and/or bed, reference in made herein to the mattresses and beds described in U.S. Patent Nos. 8,006,332; 7,690,059; 7,805,784; 7,962,981; 7,861,334; 6,843,873; 7,730,566; 7,823,233; 7,823,234; 7,827,636; 5,088,136; 5,325,551, and 5,542,136 Reference is also made to U.S. Pat. Nos. 5,749,111; 6,026,527; 6,197,099; 6,413, 458; 6,865,759; 7,060,213 and 8,075,981 for examples of suitable mattresses constructed of structural gel.

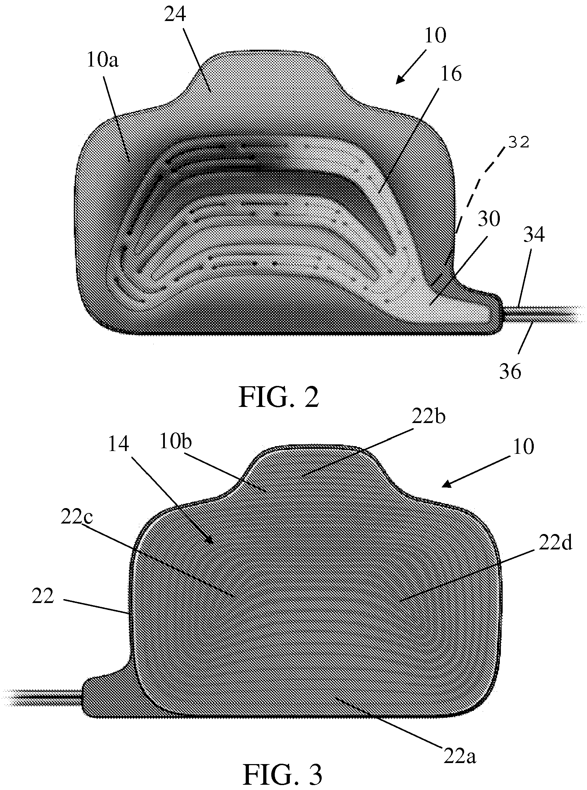

Referring to FIGS. 2 and 3, the pad 10 includes a cushioning layer 14 and one more conduits 16 for circulating fluid, such as cooled liquid including cooled water and optionally water with fluorocarbons, through pad 10 to cool a patient's skin or tissue lying on the pad. Conduits 16 are in fluid communication with a pump 18 (see FIG. 1), which circulates a fluid, such as water, through the conduits 16 in pad 10. For an example of a suitable pump, reference is a pump sold under the trademark T/PUMP® by Stryker Corporation of Kalamazoo, Mich. The pump and any supporting control system may be mounted in the mattress itself, such as described in U.S. Pat. Nos. 5,325,551, and 5,542,136, both commonly owned by Stryker Corporation of Kalamazoo, Mich., or may be located external to the mattress as shown, for example at the footboard or the side rail, or at other locations on or off the bed, including an on-board pump, such as disclosed in U.S. Patent No. 8,011,039, commonly owned by Stryker Corporation of Kalamazoo, Mich.

Cushioning layer 14 may be formed from a variety of different materials and structures to provide shear reduction and/or localized pressure management. For example, cushioning layer 14 may comprise a gel cushioning layer 20. Suitable gelatinous elastomeric materials for forming the gel layer may be formed by blending an A-B-A triblock copolymer with a plasticizer oil, such as mineral oil. The "A" component in the A-B-A triblock copolymer is a crystalline polymer like polystyrene and the "B" component is an elastomer polymer like poly(ethylene-propylene) to form a SEPS polymer, a poly (ethylene-butadyene) to form a SEBS polymer, or hydrogenated poly(isoprene + butadiene) to form a SEEPS polymer. For examples of suitable gelatinous elastomeric materials, the method of making the same, and various suitable configurations for the gel layer reference is made to U.S. Pat. Nos. 3,485,787; 3,676,387; 3,827,999; 4,259,540; 4,351,913; 4,369,284; 4,618,213; 5,262,468; 5,508,334; 5,239,723; 5,475,890; 5,334,646; 5,336,708; 4,432,607; 4,492,428; 4,497,538; 4,509,821; 4,709,982; 4,716,183; 4,798,853; 4,942,270; 5,149, 736; 5,331,036; 5,881,409; 5,994,450; 5,749,111; 6,026,527; 6,197,099; 6,843,873; 6,865,759; 7,060,213; 6,413, 458; 7,730,566; 7,823,233; 7,827,636; 7,823,234; and 7,964,664.

Other formulations of gelatinous elastomeric materials may also be used in addition to those identified in these patents. As one example, the gelatinous elastomeric material may be formulated with a weight ratio of oil to polymer of approximately 3.1 to 1. The polymer may be Kraton 1830 available from Kraton Polymers, which has a place of business in Houston, Texas, or it may be another suitable polymer. The oil may be mineral oil, or another suitable oil. One or more stabilizers may also be added. Additional ingredients such as, but not limited to, dye or microspheres may also be added. In another example, the gelatinous elastomeric material may be formulated with a weight ratio of oil to copolymers of approximately 2.6 to 1. The copolymers may be Septon 4055 and 4044 which are available from Kuraray America, Inc., which has a place of business in Houston, Texas, or it may be other copolymers. If Septon 4055 and 4044 are used, the weight ratio may be approximately 2.3 to 1 of Septon 4055 to Septon 4044. The oil may be mineral oil and one or more stabilizers may also be used. In addition to these examples, as well as those disclosed in the aforementioned patents, still other formulations may be used.

Referring to FIG. 3, in the illustrated embodiment, cushioning layer 14 includes a plurality of ribs or walls 22, which are nested and, further, are sized such that when a load, such as a patient's weight, is placed on the patient facing side of the pad 10a, the walls will buckle to prevent bottoming-out of the upper surface 24 of the pad to the upper surface of the underlying mattress. Even when fully buckled or folded, the material forming the walls will thereby provide cushioning to the patient.

Optionally, walls 22 may be arranged in closed nested loops 22a and rows 22b, with rows 22b extending between opposed edges of the pad adjacent the outermost wall of loops 22a. In the illustrated embodiment, pad 10 includes a generally rectangular shaped pad with rounded edges and with an optional enlarged lobed region 10b so that the pad focuses cooling on the sacrum, trochanter and ischium regions of the patient. As shown, rows 22 extend across the enlarged lobed region in a generally parallel relationship and are optionally evenly spaced.

As best seen in FIG. 3, loops 22a have regions of generally parallel wall sections and also include wall sections that form tightly nested regions 22c and 22d which generally correspond to the boney protruberances of the hip of a patient where the pressure exerted by a patient lying on the pad is likely to be the greatest.

Alternately, cushioning layer 14 may be formed from a foam material or may be formed from a plurality of bladders. It should be understood that the height and optionally the width of the bladders would be scaled down to fit into the pad application, where the overall height of the pad is optionally maintained at 2 inches (50.8 mm) or less, more typically 1 inch (25.4 mm) or less. Alternately, the bladders may have a tubular construction, which laterally extend across the width of the pad.

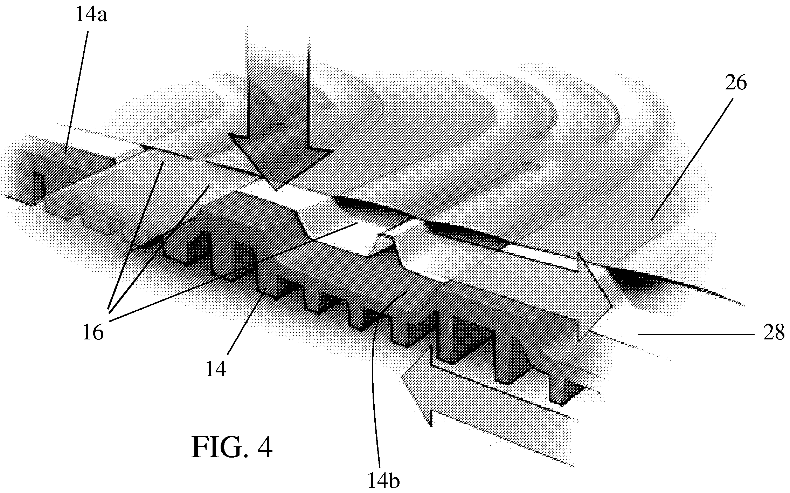

Referring to FIG. 4, cushioning layer 14 optionally includes a solid or continuous upper surface 14a that includes one or more channels 14b formed therein. Channels 14b support and located the conduits 16 in the upper surface of the cushion so that when a patient is lying on the pad, the cushion layer will support the patient rather than the conduits so that the patient's weight will not occlude the conduits, which instead will be redistributed to the downwardly facing walls of the cushioning layer.

Referring again to FIG. 4, conduits 16 are formed between two sheets of flexible material, such as two sheets of flexible film, including thermoformed plastic film, which for the patient facing side of the pad. For example, suitable films may be formed from PVC, polyurethane (PU), polyethylene (PE), polyester polyurethane (PPU) film, neoprene film, or thermapolyurethane film. Suitable polyurethane films may have a relatively low durometer, which results in the film exhibiting supple qualities and can feel very soft against human skin. Further, the elongation properties of polyurethane films are especially desirable and allow the film to significantly stretch to thereby reduce the shear stresses on a patient supported on pad 10.

For example, the upper film 26 and lower film 28 may be joined together, for example, by welding, at discrete locations to thereby form the conduit(s) 16 between the sheets. Given their elongation and flexibility characteristics, as best seen in FIG. 4, films 26 and 28 will generally follow the surface topology of upper portion 14a of cushioning layer 14 so that the conduits 16 are recessed into the channels 14b formed in upwardly facing side 14a of cushioning layer 14. Optionally when films 26 and 28 are joined together film 28 is provided with some slack or excess material so that it will balloon away from film 26 when channel 16 is filled with fluid. In this manner, even when channels 16 are inflated, upper film 26 will retain a generally flat configuration though the regions directly over the passageways may form slightly raised regions, such as shown in FIG. 4. In this manner, as described above, cushioning layer 14 will provide support to the conduits so that when a patient is lying on pad 10, the patient's weight will not occlude the conduits so that cooling fluid will continue to flow through the pad.

Referring again to FIG. 2, conduits 16 extend to the edge of pad 10, where they merge into an inlet conduit 30 and an outlet conduit 32, which are respectively in fluid communication with supply tubing 34, which extends to pump 18 for circulating the cooling fluid to the pad, and with discharge tubing 36 that returns fluid to the pump 18 for cooling and recycling back to the pad (for example in a closed loop system). Optionally, conduits 16 expand as they extend through the pad, which reduces the back pressure on the fluid as it flows through the various passageways. In some instances when dealing with heavier patients, the fluid may need to be pressurized to a higher range, and/or the size and number of the conduits and/or thickness of the pad may need to be increased to avoid occlusion of the conduits.

Alternately or in addition, pad 10 may incorporate one or more cooling components. For example, pad 10 may incorporate an electric or electronic device or a phase change material. For example, a layer of phase change material with a removable film may be applied above or adjacent the cushion layer which could be activated by removal of the film, which exposes the phase change material to air and causes the material to become cool. In another embodiment, the phase change material layer is activated by allowing an internal sack or region in the layer to break or open allowing two chemicals to combine which cause the phase change reaction. Further, the layer may be removable so that when the phase change reaction is complete or not longer generates sufficient cooling that it can be replaced.

Alternately, a semiconductor device, such as a Peltier effect device, may be embedded or applied to the upwardly facing surface, which is powered either directly through wiring or a conductive fabric or inductively powered by a transmitter adjacent the pad. When energized, the device draws energy from the interface between the patient and the pad to thereby reduce the temperature of the patient's skin cells.

In yet another embodiment, pad 10 may incorporate one or more conductive fabric layers, such as aluminum, stainless steel. silver or copper, which are in communication with one or more cooling devices, such as described above, that are located for example along the edge or end of the pad, which cool the layer(s) by conduction, which in turn then cools the interface between the patient and the pad. The silver and copper fabric then have the added benefit of providing antimicrobial properties.

As noted above, fluid conduit or the cooling component or device may be extended through pad 10 transversely across the cushioning layer to cool the patient's skin or tissue. For example, in order reduce the likelihood of damage or degree of damage to the patient's skin due to pressure exerted on the patient's skin by simply lying on a mattress, it is desirable to cool the patient's skin or tissue below 32 degrees Celsius, including temperatures below 31 degrees Celsius, below 30 degrees Celsius, below 29 degrees Celsius, below 28 degrees Celsius, below 27 degrees Celsius, below 26 degrees Celsius, and below 25 degrees Celsius. For example, to cool the skin to the above noted temperatures, the temperature of the cooled fluid may be less than 30 degrees Celsius, less than 29 degrees Celsius, less than 28 degrees Celsius, less than 27 degrees Celsius, less than 26 degrees Celsius, less than 25 degrees Celsius, less than 24 degrees Celsius, less than 23 degrees Celsius, less than 22 degrees Celsius, less than 21 degrees Celsius, or less than 20 degrees Celsius, including as low as 15 degrees Celsius. Optionally, the cooled fluid may be in a range of about 30 degrees Celsius to 15 degrees Celsius, in a range of about 28 degrees Celsius to 18 degrees Celsius, or in a range of about 26 degrees Celsius to 21 degrees Celsius.

Alternately or in addition, the cooled fluid or cooling component or device induces a minimum temperature change at the skin of the patient of about 10 degrees Celsius, of about 8 degrees Celsius, of about 5 degrees Celsius, or of about 2 or 3 degrees Celsius.

To increase the heat transfer to the fluid, the volume of fluid flowing through or the size of the cooling component or device the pad may be varied. Further, the heat transfer rate is optimally sufficient to cool the patient's skin or tissue locally and but not sufficient to cool the patient's core body temperature or at least to maintain the core body temperature within 1 to 3 degrees Celsius from the patient's normal core body temperature.

One of the goals is to cool the patient's skin sufficiently to reduce the metabolic rate of the patient's skin cells so that the need for nutrients is lowered. Another goal is to balance blood flow with the nutrient needs of the patient's skins cells. At lower temperatures, the skin cells may endure greater pressure without the attendant damage with the same pressure at higher temperatures. In other words, the lower the applied pressure, the warmer the patient's skin can be without creating tissue damage. Also, in the balance is the comfort to the patient. The cooler the skin, the more discomfort to the patient.

To monitor the heat transfer, pump 18 may include a control system with a sensor that measures the temperature of the supply fluid and a sensor that measures the discharge fluid temperature, which can be used to measure the thermal transfer and to adjust the temperature of the supply fluid if needed.

Depending on the specific application, the size of the pad may be varied. For example, for cooling the sacrum, trochanter and ischium regions the pad may have dimensions in a range of 36 (91.44 cm) to 18 (45.7 cm) in width and 24 (61 cm) to 18 (45.7 cm) in length, and have an overall thickness in a range of 2 inches (5.08 cm) to ½ inch (1.27 cm) or less.

While the pad is intended to be flexible and pliable and optionally stretchy enough to follow the surface topology of the mattress or cushion when a patient is lying on the pad, when in use, such as shown in FIG.1, pad 10 remains unfolded to prevent inadvertent occlusion of the conduits. Further, the pad is intended to be flexible and optionally stretchy enough to avoid interfering with the pressure redistribution and immersion of the patient into the mattress beneath the pad and also without generating an increase in shear on the patient's skin.

Accordingly, the pads, described above, each reduce the temperature of the skin cells of the patient by conduction. Optimally the pads, described above, each cool the skin cells of the patient sufficiently to lower the metabolic rate of the skin cells of a patient to reduce, if not eliminate, damage to the skin cells when subject to pressures associated with lying on a mattress or cushion. Further, the pad is movable and adjustable so that it can be positioned to suit the needs of a given patient.

## Claims

1. A temperature management pad comprising: a patient facing side,
a cushioning layer, said cushioning layer having a top surface forming said patient facing side and configured to support a patient on said pad and a bottom surface forming a support facing side for supporting said pad on a support surface; and
a fluid conduit formed between said patient facing side and said cushioning layer for circulating fluid through said pad across said cushioning layer and extending adjacent said patient facing side for circulating fluid across said pad to cool said patient facing side of said pad for cooling a patient lying thereon, and wherein
said cushioning layer comprises a gel cushioning layer supporting said patient facing side and said fluid conduit above the support surface ; and
wherein said gel cushioning is an elastomeric gel and has a solid upper surface, said solid upper surface having channels formed therein, and
said conduit supported in said channels for circulating fluid through said pad laterally across said cushioning layer and at said patient facing side.

2. A temperature management pad according to claim 1, wherein the cushioning layer includes a plurality of walls at said support facing side for supporting said pad on the support surface, optionally said gel cushioning layer includes gel walls.

3. A temperature management pad according to Claim 2, wherein said gel walls are configured to locally buckle under a pressure of a predetermine magnitude.

4. A temperature management pad according to Claim 3, wherein said gel walls intersect to form a grid.

5. A temperature management pad according to any above claim, wherein said gel cushioning layer comprises a structural gel

6. A temperature management pad according to any of the above claims, wherein said patient facing side includes a film, optionally, said film comprising
a flexible polymeric film, such as polyester, polyether polyurethane film, PVC film, neoprene film, or polyethylene film.

7. A temperature management pad according to Claim 6, wherein said film comprises a first film, said patient facing side further includes a second film, and said first and second films forming the boundaries of said conduit, optionally, said second film comprising a flexible polymeric film, such as polyester, polyether polyurethane film, PVC film, neoprene film, or polyethylene film.

8. A temperature management pad according to Claim 3, wherein said walls are arranged in nested loops and rows.

9. A temperature management pad according to Claim 3, wherein at least some of said loops form tightly nested regions generally corresponding to boney protruberances of the hip of a patient.

## Patentansprüche

1. Temperaturverwaltungskissen, umfassend: eine dem Patienten zugewandte Seite,
eine Polsterschicht, wobei die Polsterschicht eine obere Fläche, welche die dem Patienten zugewandte Seite bildet und ausgebildet ist, um einen Patienten auf dem Kissen zu stützen, und eine unter Fläche aufweist, die eine der Stütze zugewandte Seite zum Stützen des Kissens auf einer Stützfläche bildet; und
eine Fluidleitung, die zwischen der dem Patienten zugewandten Seite und der Polsterschicht gebildet ist, um Fluid durch das Kissen über die Polsterschicht zu zirkulieren, und die sich benachbart zu der dem Patienten zugewandten Seite erstreckt, um Fluid über das Kissen zu zirkulieren, um die dem Patienten zugewandte Seite des Kissens zu kühlen, um einen Patienten zu kühlen, der darauf liegt, und wobei
die Polsterschicht eine Gel-Polsterschicht umfasst, welche die dem Patienten zugewandte Seite und die Fluidleitung über der Stützfläche stützt; und
wobei das Gel-Polster ein Elastomergel ist und eine feste Oberfläche aufweist, wobei die feste Oberfläche darin gebildete Kanäle aufweist, und
die Leistung in den Kanälen gestützt wird, um Fluid durch das Kissen seitlich über die Polsterschicht und auf der dem Patienten zugewandten Seite zu zirkulieren.

2. Temperaturverwaltungskissen nach Anspruch 1, wobei die Polsterschicht eine Vielzahl von Wänden an der der Stütze zugewandten Seite beinhaltet, um das Kissen auf der Stützfläche zu stützen, wobei die Gel-Polsterschicht gegebenenfalls Gel-Wände beinhaltet.

3. Temperaturverwaltungskissen nach Anspruch 2, wobei die Gel-Wände ausgebildet sind, um lokal einem Druck einer vorbestimmten Stärke nachzugeben.

4. Temperaturverwaltungskissen nach Anspruch 3, wobei sich die Gel-Wände schneiden, um ein Netz zu bilden.

5. Temperaturverwaltungskissen nach einem der vorangehenden Ansprüche, wobei die Gel-Polsterschicht ein Strukturgel umfasst.

6. Temperaturverwaltungskissen nach einem der vorangehenden Ansprüche, wobei die dem Patienten zugewandte Seite einen Film beinhaltet, wobei der Film gegebenenfalls eine flexible Polymerfolie umfasst, wie etwa Polyester, Polyetherpolyuretanfolie, PVC-Folie, Neoprenfolie oder Polyethylenfolie.

7. Temperaturverwaltungskissen nach Anspruch 6, wobei die Folie eine erste Folie umfasst, wobei die dem Patienten zugewandte Seite ferner eine zweite Folie umfasst und die erste und zweite Folie die Begrenzungen der Leitung bilden, wobei die zweite Folie gegebenenfalls eine flexible Polymerfolie umfasst, wie etwa Polyester, Polyetherpolyuretanfolie, PVC-Folie, Neoprenfolie oder Polyethylenfolie.

8. Temperaturverwaltungskissen nach Anspruch 3, wobei die Wände in verschachtelte Schleifen und Reihen angeordnet sind.

9. Temperaturverwaltungskissen nach Anspruch 3, wobei zumindest einige der Schleifen eng verschachtelte Regionen bilden, die im Allgemeinen knochigen Vorsprüngen der Hüfte eines Patienten entsprechen.

## Revendications

1. Protection de prise en charge thermique comprenant : un côté en regard du patient,
une couche d'amortissement, ladite couche d'amortissement présentant une surface supérieure formant ledit côté en regard du patient et configurée pour supporter un patient sur ladite protection et une surface inférieure formant un côté en regard du support pour supporter ladite protection sur une surface de support ; et
une conduite de fluide formée entre ledit côté en regard du patient et ladite couche d'amortissement pour faire circuler un fluide dans ladite protection à travers ladite couche d'amortissement et s'étendant de manière adjacente audit côté en regard du patient pour faire circuler un fluide à travers ladite protection pour refroidir ledit côté en regard du patient de ladite protection pour refroidir un patient allongé sur celle-ci, et dans laquelle
ladite couche d'amortissement comprend une couche d'amortissement en gel supportant ledit côté en regard du patient et ladite conduite de fluide au-dessus de la surface de support ; et
dans laquelle ledit gel d'amortissement est un gel élastomère et présente une surface supérieure solide, ladite surface supérieure solide présentant des canaux formés dans celle-ci, et
ladite conduite supportée dans lesdits canaux pour faire circuler le fluide dans ladite protection latéralement à travers ladite couche d'amortissement et au niveau dudit côté en regard du patient.

2. Protection de prise en charge thermique selon la revendication 1, dans laquelle la couche de protection inclut une pluralité de parois au niveau dudit côté en regard du support pour supporter ladite protection sur la surface de support, optionnellement ladite couche d'amortissement en gel inclut des parois en gel.

3. Protection de prise en charge thermique selon la revendication 2, dans laquelle lesdites parois en gel sont configurées pour se déformer sous une pression d'une force prédéterminée.

4. Protection de prise en charge thermique selon la revendication 3, dans laquelle lesdites parois en gel se croisent pour former un treillis.

5. Protection de prise en charge thermique selon une quelconque revendication précédente, dans laquelle ladite couche de protection en gel comprend un gel structurel.

6. Protection de prise en charge thermique selon l'une quelconque des revendications précédentes, dans laquelle ledit côté en regard du patient inclut un film, optionnellement, ledit film comprenant un film polymère souple, comme du polyester, un film de polyuréthane de polyéther, un film PVC, un film néoprène, ou un film polyéthylène.

7. Protection de prise en charge thermique selon la revendication 6, dans lequel ledit film comprend un premier film, ledit côté en regard du patient inclut en outre un deuxième film, et lesdits premier et deuxième films formant les limites de ladite conduite, optionnellement, le deuxième film comprenant un film polymère souple, comme du polyester, un film de polyuréthane de polyéther, un film PVC, un film néoprène, ou un film polyéthylène.

8. Protection de prise en charge thermique selon la revendication 3, dans laquelle lesdites parois sont agencées en rangées et boucles imbriquées.

9. Protection de prise en charge thermique selon la revendication 3, dans laquelle au moins certaines desdites boucles forment des régions étroitement imbriquées correspondant généralement à des protubérances osseuses de la hanche d'un patient.
